# EUROPEAN PATENT APPLICATION

(11) **EP 2 236 597 A1**
(43) Date of publication of application: **06.10.2010**
(21) Application number: 10003330.7
(22) Date of filing: 29.03.2010
(51) Int. Cl.: C12M 3/00

(54) **High-throughput sensorized bioreactor for applying hydrodynamic pressure and shear stress stimuli on cell cultures**

(30) Priority: 30.03.2009 IT PI20090033
(71) Applicant: UNIVERSITA' DI PISA, 56126 Pisa (IT)
(72) Inventor: Ahluwalia, Arti, 54035 Fosdinova (Massa-Carrara) (IT); De Maria, Carmelo, 88046 Lamezia Terme (Catanzaro) (IT); Mazzei, Daniele, 56122 Pisa (IT); Vozzi, Giovanni, 56124 Pisa (IT)
(74) Representative: Lunati & Mazzoni S.r.L.

(57) **Abstract**

A bioreactor (1) for subjecting a cell culture (3) to a hydrodynamic pressure stimulus is described. This hydrodynamic pressure is generated inside at least one culture chamber (2) by means of variation of the mean distance, with a controlled speed, of a surface relative to the surface on which the culture (3) is positioned and between which the culture medium (5) is free to flow.

## Description

The present invention relates to a high-throughput sensorized bioreactor for the application of hydrodynamic pressure and shear stress on cell cultures, tissue constructs or tissues of the type pointed out in the preamble of claim 1.

In particular, it relates to a high-throughput sensorized bioreactor that can be placed in series and in parallel to other similar devices, through which cell cultures of one or more types are subjected to mechanical stimuli such as hydrodynamic pressure and shear stress, thereby simulating the physiological or pathological conditions present inside an organism.

The applications of this bioreactor fall within the sector of Tissue Engineering, and are aimed to promote cell proliferation and differentiation for the development of functional biological constructs.

All tissues or biological systems are subjected to physical and chemical stimuli during their development and normal activity, and these determine their physiopathological state and affect their normal function.

In particular, each organism is constantly subjected both to external mechanical loads such as gravity and movement, and to inner forces such as contractile and hemodynamic forces, mainly generated by muscle cells. Each cell, due to its cytoskeleton, has the possibility of developing and sensing external forces affecting the morphology, cytoskeletal organisation, survival, cellular differentiation and gene expression.

In this context, the ExtraCellular Matrix, commonly referred to as ECM, plays a fundamental role, and it's characterised by a bi-directional interaction with cells, i.e. chemical, mechanical or electric variations in the ECM induce cellular modification that can result in modifications of the external environment through the synthesis of ECM components.

The mechanical transduction mechanisms have not been yet well understood, i.e. those mechanisms whereby a mechanical force is converted into a chemical and biochemical signal capable of triggering signal transduction pathways and modulating gene expression. For this reason many studies have been carried out obtaining a satisfactory approximation of said mechanisms.

Tissue Engineering of ECM and therefore of mechanically functional 3D tissues, such as bone, cartilage and muscle, is approached by manipulating four main variables: the cell type, the scaffold, the biochemical factors (peptides and growth factors, for example), and the mechanical forces.

For reproducing the physico-chemical stimuli in cell cultures or on tissue explants, bioreactors have been developed which are able to simulate the mechanobiological environment present inside an organism.

In fact, bioreactors for the application of mechanical stresses on different types of cells and tissues are known.

An example of these bioreactors is the 2-D culture system based on a flexible membrane which is used for examining the cyclical strain effects on cell monolayers placed directly on the membrane or in combination with another substrate.

A further example is given by the rotary 3-D bioreactor, the so-called "Rotary Cell Culture System", which has been used for increasing the uniformity of seeding on 3-D scaffolds and simulating microgravity environments.

Another example of a bioreactor for linear cyclic traction of tissue constructs can be found in the work by Webb (Webb K, Hitchcock RW, Smeal RM, Li W, Gray SD, Tresco PA, Cyclic strain increases fibroblast proliferation, matrix accumulation, and elastic modulus of fibroblast-seeded polyurethane constructs, J Biomech. 2006; 39(6): 1136-44). They obtained an increase in fibroblast proliferation, a greater ECM production and an increase in the elastic modulus of the fibroblast-seeded polyurethane construct. Similar effects have been also obtained on osteoblastic cells (Ignatius A, Blessing H, Liedert A, Schmidt C, Neidlinger-Wilke C, Kaspar D, Friemert B, Claes L. Tissue engineering of bone: effects of mechanical strain on osteoblastic cells in type I collagen matrices. Biomaterials. 2005 Jan; 26(3):311-8).

Also belonging to this category is the US patent 20040219659 describing a device for translational and rotational strain of bioengineered tissues, with a simultaneous flow of culture medium through the tissue and/or the culture cell itself.

There are many mechanical solutions conceived for recreating the stresses of normal articulation *in vitro*: systems for both static and dynamic compression loads (J Steinmeyer. A computer-controlled mechanical culture system for biological testing of articular cartilage explants. J Biomech, 30(8):841-5, Aug 1997. Twana Davisson, Sabine Kunig, Albert Chen, Robert Sah, and Anthony Ratcliffe. Static and dynamic compression modulate matrix metabolism in tissue engineered cartilage. J Orthop Res, 20(4):842-8, Jul 2002), for bending stress (M T De Witt, C J Handley, B W Oakes, and D A Lowther. In vitro response of chondrocytes to mechanical loading, the effect of short term mechanical tension. Connect Tissue Res, 12(2):97-109, 1984. M O Wright, K Nishida, C Bavington, J L Godolphin, E Dunne, S Walmsley, P Jobanputra. G Nuki, and D M Salter. Hyperpolarisation of cultured human chondrocytes following cyclical pressure-induced strain: evidence of a role for alpha 5 beta 1 integrin as a chondrocyte mechanoreceptor. J Orthop Res, 15(5):742-7, Sep 1997) and hydrostatic pressure systems (Shuichi Mizuno, Tetsuya Tateishi, Takashi Ushida, and Julie Glowacki. Hydrostatic fluid pressure enhances matrix synthesis and accumulation by bovine chondrocytes in three-dimensional culture. J Cel I Physiol. 193(3):319-27, Dec 2002, R L Smith, J Lin, M C Trindade, J Shida, G Kajiyama, T Vu, A R Hoffman, M C van der Meulen, S B Goodman, D J Schurman, and D R Carter. Time-dependent effects of intermittent hydrostatic pressure on articular chondrocyte type II collagen and aggrecan mrna expression. J Rehabil Res Dev, 37(2):153-61-2000).

The work by Frank (Frank EH, Jin M. Loening AM, Levenston ME, Grodzinsky AJ. A versatile shear and compression apparatus for mechanical stimulation of tissue culture explants. J Biomech. 2000 Nov; 33(11):1523-7) is distinguishable from the previously described works because in this work a bioreactor for chondrocytes capable of carrying out biaxial shear (resolution 0.01°) and compression (resolution 1 µm) strains is described.

The bioreactor described in patent US20020106625 developed for generating a functional cartilage construct, simultaneously applies a hydrostatic pressure and a strain through a load plate, whereas the device described in patent US20060068492, in addition to applying a compression strain through a load plate, submits the test piece to shear stresses through a rotary motion of the culture chamber.

Finally, devices such as the "Flat Bed Perfusion System" or the "laminar flow bioreactor", due to a flow of the culture medium through the culture chamber, enable a greater flow of metabolites and allow endothelial cells to be submitted to shear stress due to the shear rate of the perfused media. Finally, 3-D bioreactors with a pulsatile flow are used for inducing smooth muscle cell alignment for engineering blood vessels.

The known art mentioned above has some important drawbacks.

In fact, most of known bioreactors are not autonomous, because they need an incubator for ensuring the required pH and temperature values inside the culture chamber.

The need for an incubator and/or the lack of an appropriate sensor-based system restricts the use of computers that could enable the variation of experimental parameters be monitored and controlled in real time during the experiment or test. None of the bioreactors present on the market and/or described in the literature generates hydrodynamic pressure; some systems limit themselves to stimulate the cell cultures with hydrostatic pressure generated through pressure regulators or other hydraulic mechanisms.

In this context, the technical task underlying the present invention is to devise a high-throughput sensorized bioreactor for the application of hydrodynamic pressure and shear stress on cell cultures that is capable of substantially obviating the mentioned drawbacks.

Hydrodynamic pressure stimulation appears to be much more similar to that present inside articular joints, wherein an overpressure is developed by the mutual movement of the bone-ends.

Another aim of the invention is to enable the use of cell lines or tissue explants, which will therefore reduce animal testing.

Another important aim of the invention is to provide a bioreactor enabling a precise evaluation of the biological processes in different types of cells submitted to suitable chemical and/or physical stimuli which recreate the physiopathological stimuli present *in-vivo*.

Furthermore it is not of minor importance to succeed in obtaining a reduction in costs and testing times.

Therefore, another aim of the bioreactor is to minimize of the volume of culture medium enabling biochemical analyses to be carried out without expensive procedures for concentrating the solutions.

The technical task mentioned and the aims specified are achieved by creation of a hydrodynamic pressure that can be obtained by means of the mutual movement of rigid surfaces between which a fluid is free to flow. In this manner a pressure gradient is generated that further determines a flow between the two mutually moving surfaces. In addition, the shear stress due to the shear rate of this flow gives rise to a further stimulus on the cultured construct.

With this aim, the inventors have sought to develop bioreactors with culture chambers using limited culture medium volumes and capable of reproducing a physical stimulus of hydrodynamic pressure and shear stress in addition to other mechanical and/or chemical stimuli. These culture chambers can be connected in series or in parallel to reproduce metabolic processes of organ systems or biological tissues and are provided with sensors suitable for the study of the influence of these stimuli on cell function.

Therefore, in the present invention a bioreactor has been developed which stimulates the mechanical environment present *in-vivo* by means of the generation of a localised pressure utilising the principle of hydrodynamic lubrication.

In conclusion, the invention described is a device acting as a bioreactor for the culture of cell constructs and/or tissue explants, which is independent of an incubator (free-standing), with limited culture medium volumes, whose culture chambers, made of biocompatible and easily sterilisable materials, can be connected to each other, in series and/or in parallel, and form combinations thereof, through a perfusion circuit. Within these chamber the cultures, i.e. the cell constructs and/or tissue explants, can be subjected to different physiopathological environments simulated by means of physico-chemical stimuli. In particular the pressure and shear stresses can be obtained through movement of a moving portion within the culture medium according to the hydrodynamic pressure-generating mechanisms described in the following.

These and other stimuli will be produced by a set of devices present in the culture chamber or along the perfusion circuit, controlled by a suitable local programmable hardware managed by a PC through suitably developed software.

In addition, the state of the experiment or test in progress within this new bioreactor device will be controlled in real time through suitable physico-chemical sensors present within the culture chamber and in the perfusion circuit. These sensors will be managed by the same hardware-software system mentioned above.

In particular, in the present invention a bioreactor is developed which simulates the mechanical environment present *in-vivo* through generation of a localised pressure utilising the hydrodynamic lubrication principle.

Generation of a hydrodynamic pressure in a fluid interposed between two bodies can only take place if the two bodies are in relative motion.

The pressure field in the interspace between the two bodies (meatus) can be described by the Reynolds equation which derives from the combination of the force equilibrium and continuity equations, also using the constitutive and congruency equations. In particular, an in-depth description of the Reynolds equation and of said pressure can be found in "*Principles and applications of tribology*" by Desmond F. Moore, published in 1975.

By virtue of the overpressure generated, it is also possible to give rise to a local and controlled flow through which the cultured cells are stimulated, with a shear stress due to the shear rate of the fluid.

In practise, this intervention allows a high-throughput battery of experiments to be performed simultaneously. This feature, as can be inferred, allows: a reduction in animal experiments and consequently in animal suffering because only cell lines or tissue explants are used; a precise evaluation of the biological process of the different cell types submitted to suitable chemical and/or physical stimuli that recreate the physiopathological stimuli present *in vivo*; a reduction in the testing costs and times.

The technical task mentioned and the aims specified are achieved by a high-throughput sensorized bioreactor for the application of hydrodynamic pressure and shear stress on cell cultures as claimed in the appended Claim 1.

Preferred embodiments are highlighted in the sub-claims.

The features and advantages of the invention are hereinafter clarified by the detailed description of some preferred embodiments of the invention, with reference to the accompanying drawings, in which:
**Fig. 1** diagrammatically shows a schematic of the geometry of the bioreactor on which the mathematical model of one of the preferred embodiments is based for generating hydrodynamic pressure;
**Fig. 2** shows the whole high-throughput bioreactor 1 according to the invention;
**Fig. 3** shows a portion of the bioreactor in a first embodiment;
**Fig. 4a** shows a section of a part according to the first embodiment;
**Fig. 4b** is a sectional view of the same part, but according to a second embodiment; and
**Fig. 4c** reproduces a sectional view of the same part, but according to a third embodiment.

With reference to the mentioned figures, the high-throughput sensorized bioreactor for application of hydrodynamic pressure and shear stress on cell cultures, tissue constructs or tissues according to the invention is generally identified with the reference number **1.**

It comprises: at least one culture chamber **2,** adapted to accommodate a culture or a natural/artificial cellular construct **3** and having an inlet port **2a** and an outlet port **2b** for a fluid culture medium **5;** a mixing chamber **4** separated from the culture chamber/s 2 and suitable to prepare the said medium 5; a perfusion circuit of the culture chamber 2 connected to said inlet 2a and outlet 2b ports and comprising said mixing chamber 4; flow circuit **6** for producing a controlled flow of the culture medium 5, through said circuit; at least one stimulating apparatus **7** for producing physico-chemical stimuli, which is disposed at least within the culture chamber 2 and, preferably, also in the aforesaid circuit; and control devices **8** for adjustment of the physico-chemical parameters, such as temperature, pH, overpressure for example, and of all the other physico-chemical stimuli that are wished to be applied to the culture under examination.

In particular, herein the term "culture" indicates a natural/artificial cell construct or a monolayer.

All the above elements can be managed by a suitably developed software that, by a user friendly graphic interface, allows the experimental parameters to be set and modified in real time and the state of the cell environment to be viewed in real time.

In particular, a plurality of culture chambers 2 can be present, which are connected in series and/or in parallel to each other through suitable channels so as to simulate the behaviour of complex biological organism. In greater detail, different culture chambers 2 can be analysed simultaneously in a single bioreactor 1, by virtue of said plurality of chambers 2.

In addition, the inlet 2a and outlet 2b ports are suitably positioned in such a manner so as not to interfere with the mechanical stimulation and so that the local overpressure does not affect the recirculation of the culture medium 5.

The culture chamber 2 is made of a biocompatible material following suitable geometries ensuring all the above mentioned features. It can be redesigned each time using software for mechanical design.

Advantageously, said chamber 2 can have a preferred direction of extension **2c** and in particular a tubular shape. This direction, even in those embodiments in which it is not the preferred direction of extension, is referred to and denoted with the abbreviation 2c.

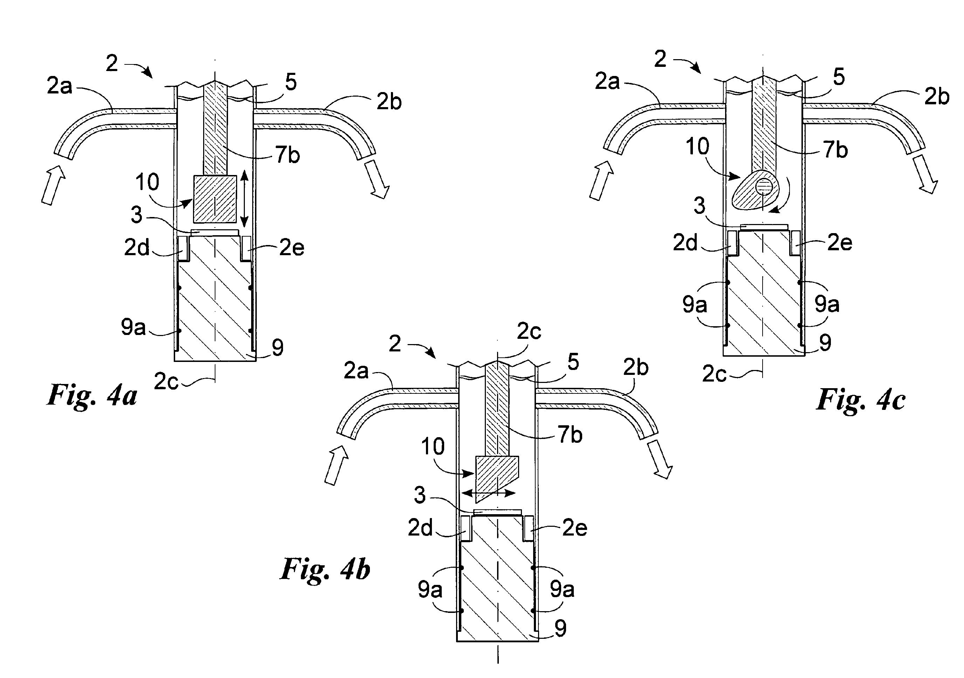

As shown in Figs. 4a, 4b and 4c, it can be provided with a system of integrated or non-integrated sensors for analysing the vitality and functions of cells in the culture chamber 2 in real time. In particular, a chamber 2 can be suitably provided with physical **2d** and/or chemical **2e** integrated sensors that can be obtained following macro-fabrication, micro-fabrication and/or nano-fabrication techniques. In particular, the physical sensors measures physical parameters such as temperature, and the chemical sensors measure the pH or other chemical parameters.

Finally, a suitable closing element **9** is secured in a fixed manner to the lower end of this culture chamber 2 and the culture 3 is disposed thereon; in particular, culture 3 is fixedly and rigidly connected to the closing element 9 and, therefore, to chamber 2 as well. The element 9 is further adapted to be coupled and sealed to the chamber 2. In particular, said coupling can be obtained using a threaded or plug-based system and preferably can advantageously involve the use of seals **9a.**

In a suitable manner, a proper closure, not shown in the figure, is provided at the other end of the culture chamber and it allows for the movement of the stimulating apparatus 7 inside the chamber 2. In this case too, the hermetic tightness can be ensured by suitable seals 9a.

The stimulating apparatus 7 comprises at least one moving component **10** at least partly disposed in chamber 2 so as to vary, preferably in a continuous manner, the mean distance along the direction 2c between the culture 3 and the component itself; and a motor **11** to move said component 10. In addition, the stimulating apparatus 7 can suitably include an appropriate supporting structure **7a** adapted to enable housing of the culture chamber 2 and of the constituent elements of the stimulating apparatus 7; and a mechanism **7b** which for example can consist of shafts, belts, gears or any other kinematic mechanism which can transmit motion between motor 11 and the moving component 10.

In particular, by the above term "mean distance along the direction 2c" it is intended the minimum distance between the centre of gravity of culture 3 and a point defined by the intersection of component 10 and a straight line parallel to the direction 2c and passing through the centre of gravity of culture 3.

In addition, in order to create a hydrodynamic pressure, the moving component 10 is preferably adapted not to take up the whole inner section of the culture chamber 2. In greater detail, the dimensions of component 10 are of such a nature that a free movement is allowed to the culture medium 5 present in chamber 2 during the movement of component 10. In particular, the medium 5 can flow between the culture chamber 2 and the moving component 10, as shown in Figs. 4a, 4b and 4c.

In fact, should the moving component 10 have sizes equal to the inner diameter of the culture chamber 2, movement of component 10 would give rise to a hydrostatic pressure, due to compression of the medium 5, that would not guarantee an acceptable result.

To conclude, the moving component 10 is located and moved in such a manner that at least for part of its motion it enters the culture medium 5 and is therefore capable of moving the medium itself giving rise to creation of hydrodynamic pressure. In particular, the hydrodynamic pressure thus created exerts pressure on the culture 3 and, more precisely exerts a pressure with a direction almost parallel to direction 2c. This hydrodynamic pressure is a pressure due to the relative motion between two components, in particular between the moving component 10 and the culture 3.

A first example of a stimulating apparatus 7 shown in Fig. 4a contemplates that the moving component 10 be made up of a piston the head of which has a smaller diameter than the inner diameter of the culture chamber 2, so as to enable a free movement of the piston itself and, in particular, to ensure flow of the medium 5 between the chamber 2 and the piston.

Finally, in this particular case, the piston is preferably moved by the motor in a direction substantially parallel to the extension direction 2c thus varying the above defined mean distance, i.e. the distance between the piston and culture 3 along the direction 2c in a continuous manner.

In a second example, the moving component 10 is made up of a prism having a trapezoidal base, whose extension axis is substantially perpendicular to direction 2c. In particular, said component is at least partly disposed within the culture chamber 2 with the inclined face facing the culture 3, as shown in Fig. 4b.

The prism is finally moved in a direction almost perpendicular to the direction 2c and the extension axis of the prism, enabling the mean distance between the component 10 and the culture 3 to be varied in a continuous manner, thus determining the hydrodynamic pressure.

Alternatively, as shown in Fig. 4c, the moving component consists of a cam, i.e. an element adapted to eccentrically rotate about an axis. In particular, the cam is moved by the motor and, more precisely, is set in rotation around said axis that is preferably perpendicular to the direction 2c.

Therefore, by virtue of the particular shape of the cam, said cam is adapted to create a hydrodynamic pressure acting on the culture itself. In particular, it is adapted to move close to and away from culture 3 varying the above defined mean distance in a continuous manner, i.e. the distance between the cam and culture 3 along the direction 2c.

The flow circuit 6 comprises at least one of the following elements: a fluidic system **12** brings the culture chamber 2 into communication with each other and with the mixing chamber 4 for fluid passage; a peristaltic pump **13** installed along said fluidic system 12 which ensures the correct flow direction of the culture medium 5; at least one withdrawal/admission point **14** to enable sampling of the culture medium 5 or introduction of possible drugs or other substances into the system 12 with the purpose of increasing or inhibiting cellular activities upstream of a chamber 2.

In particular, these withdrawal/admission points 14 are suitably disposed in the vicinity of the culture chamber 2 and, more precisely, downstream and upstream of each chamber 2.

The mixing chamber 4 comprises the following components not shown in the figure: a container of inert material which contains a part of the medium 5; a cup of inert material; and preferably measurement devices for monitoring the physiological parameters of the medium 5.

Advantageously, said measurement devices can comprise at least one of the following elements: a pH sensor dipped into the medium 5 present in said mixing chamber 4; a temperature sensor for measurement inside said mixing chamber 4; and sensors for measuring chemical species such as O₂, CO₂, NO, etc.

Advantageously, the inner topology of the mixing chamber 4 is planned in such a manner that positioning of the pH sensor is suitable to protect it from the direct contact with possible gas bubbles introduced into said mixing chamber 4 for balancing the medium 5 and maintain it at a desired pH.

Finally, the mixing chamber 4 can be suitably provided with valves, i.e. solenoid valves, to regulate the flow entering and coming out of said chamber.

The control devices 8 preferably comprise at least one of the following elements: a fluid flow regulated by a thermostat in a duct surrounding said mixing chamber 4 or heating systems based on Peltier cells or thermoresistors connected to and controlled by an electronic temperature regulator; inlet/outlet ports for introduction and discharge of gas, air and CO₂ for example, into/from said mixing chamber for varying the pH thereof.

Finally, the control devices 8 can advantageously comprise apparatus for monitoring and controlling the physico-chemical stimuli applied to culture 3 in the culture chamber 2. They can comprise at least one of the following components: an optical sensor for detection of bubbles inside the culture chamber 2; sensors for detection of strain and mechanical forces; sensors for detection of pressure and flow; integrated and micro-fabricated biosensors enabling the release or consumption of biomolecules of interest to be monitored; and systems for application of electrical and/or mechanical stimuli.

Preferably, the bioreactor 1 is suitable to be connected to an electronic device **15** adapted to amplify and filter the electric signals from the sensors for measuring said physiological parameters of the culture medium 5, as well as for command of the solenoid valves and application of the different electric and/or mechanical stimuli.

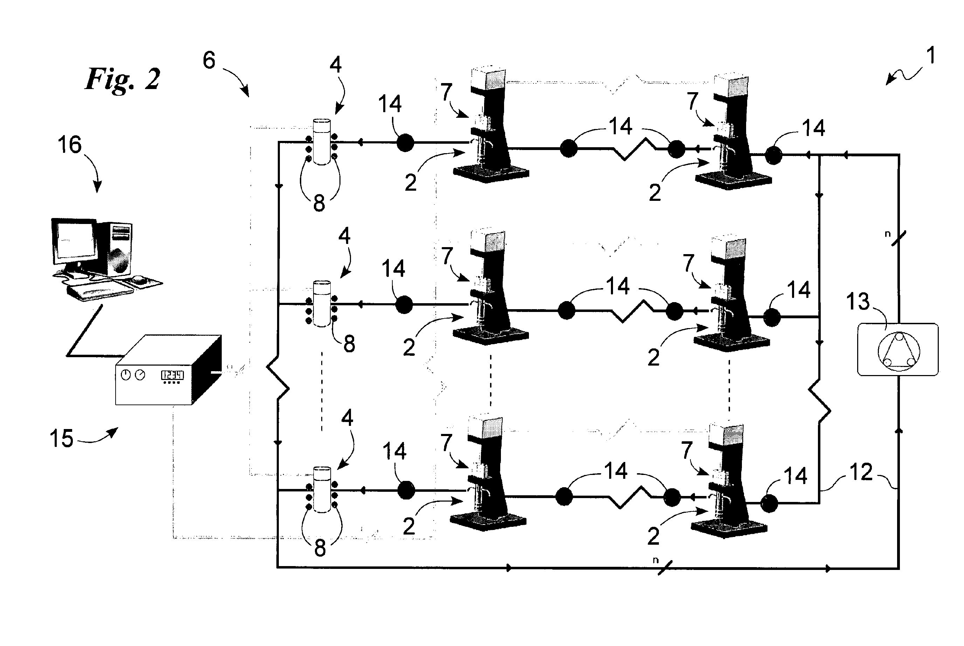

In particular, said device 15 is interfaced with a computer **16** allowing use of a software for control and management of the bioreactor 1 and therefore enabling setting and control in real time of the environment in culture chamber 2 allowing the parameters to be adjusted depending on the desired specifications. In addition, as shown in Fig. 2, computer 16 and, in particular, the electronic device 15 are suitably connected to at least part of the elements constituting the bioreactor 1 through electric cables **15a** or other means adapted to ensure said connection.

Finally, the bioreactor 1 can be alternatively introduced into an incubator, in which case the task of maintaining the environmental parameters such as pH and temperature is performed by the incubator itself without a direct control by the operator.

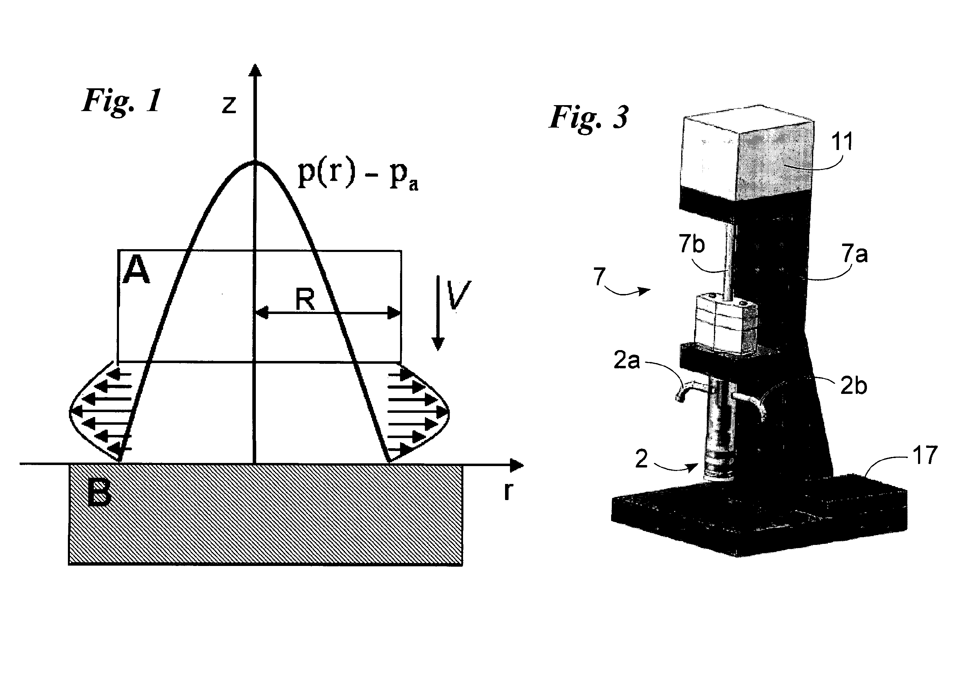

In addition, as shown in Fig. 3, in the vicinity of each chamber a local hardware **17** can be appropriately disposed, a microcontroller for example, for management of the experiment and acquisition of the signals. This hardware 17 can be connected through wireless or by a cable to the electronic device 15 or, alternatively, directly to computer 16. Finally it can regulate the motion of the moving element 10.

The procedure to use the high-throughput sensorized bioreactor for application of hydrodynamic pressure and shear stress on cell cultures, whose structure is described above, is as follows.

At the beginning culture 3 is disposed on the closing element 9 and, more precisely, is secured to the top of said element 9 which is then fixed to the culture chamber 2, as shown in Figs. 4a, 4b and 4c.

The mixing chamber 4 prepares the culture medium 5 to be used for feeding the culture chamber 2. Simultaneously, the control devices 8 regulate the parameters of the medium 5, such as pH and temperature. Finally, the values of these parameters are transmitted to the electronic device 15 and then sent to the computer 16 enabling control of said parameters in real time.

When preparation of medium 5 has been completed, the peristaltic pump 13 is activated and it sets in motion the culture medium 5 that is drawn out of the mixing chamber 4 and brought to the culture chamber 2 through the fluidic system 12.

In addition, if necessary, further biomolecules or other substances are introduced into the culture medium 5 through the withdrawal/admission points 14. Alternatively, samples of the culture medium 5 are taken for carrying out an analysis of same and therefore monitoring the physico-chemical properties of the medium.

After the medium 5 has reached the inside of chamber 2 and filled it at least partly, the stimulating apparatus 7 is activated and more specifically the moving component 10 is set in motion. In particular, component 10 varies the mean distance, as previously defined, relative to culture 3 determining the motion of medium 5 and therefore a hydrodynamic pressure adapted to exert at least one action nearly parallel to the direction 2c.

In conclusion, due to the advantageous presence of apparatus 7, a hydrodynamic pressure is generated that mimics a real situation ensuring a high quality of the results. In particular, this pressure is generated by the moving component 10 the specific geometry of which and the particular movement make the medium 5 flow between the cell 2 and the component itself thus generating the desired hydrodynamic pressure.

In conclusion, the described operations constitute a step of the stimulation to which one or more cultures are simultaneously submitted. In particular, this stimulation can consist of several steps in succession, which can differ from each other for duration or for the stimulation conditions such as the intensity of the hydrodynamic pressure, temperature, pH, characteristics of the culture medium 5.

When the stimulation has been completed, apparatus 7 is stopped and the closing element 9 is removed, the culture 3 is taken out of chamber 2.

The invention allows has important advantages.

In particular, the bioreactor 1 is adapted to simulate a mechanical environment present *in-vivo* by a generation of a localised hydrodynamic pressure.

A further advantage is represented by the use of smaller volumes of both the culture medium 5 and culture 3. Furthermore, the analysis is much quicker as compared with those carried out with other bioreactors.

Therefore the bioreactor 1 is suitable for quick and cheaper analyses.

A further advantages of bioreactor 1 consists in the possibility of using cellular lines or tissue explants and therefore reducing animal experiments.

## Claims

1. A bioreactor (1) for subjecting a culture (3) to a hydrodynamic pressure, this hydrodynamic pressure being generated inside at least one culture chamber (2) by means of the variation of the mean distance, with controlled speed, of a surface relative to the surface on which said culture or natural/artificial cell constructs (3) are positioned and between which a culture medium (5) is free to flow.

2. A bioreactor (1) as claimed in claim 1, comprising:
- at least one of said culture chamber (2) that could have a preferred direction of extension (2c) and suitable to be partly filled with said fluid culture medium (5),
- at least one culture (3) rigidly connected to said culture chamber (2) and suitable to be at least partly dipped in said culture medium (5),
- at least one stimulating apparatus (7) comprising at least one moving element (10) which can be moved within said culture chamber (2)
- **characterised in that**
said stimulating apparatus (7) can vary said mean distance along the direction (2c) of said moving element (10) from said culture (3) and wherein said culture medium (5) can flow between said moving element (10) and said culture chamber (2).

3. A bioreactor (1) as claimed in claim 2, wherein said movement of said moving element (10) can generate said hydrodynamic pressure.

4. A bioreactor (1) as claimed in the preceding claims 2-3, wherein said hydrodynamic pressure generates a motion of the culture medium (5) suitable to determine a controlled shear stress on said culture (3).

5. A bioreactor (1) as claimed in one or more of the preceding claims 2-4, wherein said moving element (10) consists of a piston.

6. A bioreactor (1) as claimed in one or more of the preceding claims 2-4, wherein said moving element (10) consists of a prism having a trapezoidal base.

7. A bioreactor (1) as claimed in one or more of the preceding claims 2-4, wherein said moving element (10) consists of a cam.

8. A bioreactor (1) as claimed in one or more of the preceding claims 2-7, can be interfaced with a local hardware (17) for setting and modifying the motion of each of said moving elements (10) in real time.

9. A bioreactor (1) as claimed in one or more of the preceding claims 2-8, wherein each of said culture chamber (2) is provided with at least one inlet port (2a) and one outlet port (2b) adapted to enable said culture medium (5) to enter and come out of said culture chamber (2) and wherein said inlet (2a) and outlet (2b) ports do not affect said hydrodynamic pressure.

10. A bioreactor (1) as claimed in one or more of the preceding claims 2-9, comprising a mixing chamber (4), for to preparing said culture medium (5) and control devices (8) for adjustment of physico-chemical parameters.

11. A bioreactor (1) as claimed in claim 10, wherein said control devices (8) can adjust the temperature and pH.

12. A bioreactor (1) as claimed in one or more of the preceding claims 10-11, comprising a plurality of said chambers (2) brought in communication with each other and with said mixing chamber (4) for fluid passage, by means of a channel system (12).

13. A bioreactor (1) as claimed in one or more of the preceding claims 2-12, comprising at least one withdrawal/admission point (14) adapted to enable both taking a sample of said culture medium (5) and introduction of substances into said culture medium (5).

14. A bioreactor (1) as claimed in one or more of the preceding claims 2-13, wherein at least one of said culture chambers (2) is provided with physical sensors (2d) for evaluating physical parameters of said culture chamber (2) and with chemical sensors (2e) for evaluating chemical parameters of said culture chamber (2).

15. A bioreactor (1) as claimed in one or more of the preceding claims 2-14, wherein each of said culture chambers (2) is provided with one of said stimulating apparatus (7).
